Europäisches Patentamt

(19) European Patent Office

Office européen des brevets

(11) Publication number : **0 498 612 A1**

## (12) EUROPEAN PATENT APPLICATION

(21) Application number : **92300925.2**

(22) Date of filing : **04.02.92**

(51) Int. Cl.⁵ : **A61F 13/62**

(30) Priority : **05.02.91 US 650927**

(43) Date of publication of application :
**12.08.92 Bulletin 92/33**

(84) Designated Contracting States :
**AT BE CH DE DK ES FR GB GR IT LI LU MC NL
PT SE**

(71) Applicant : **Beplate, Douglas K.**
**6688 Royal Harvest Way No. 11**
**Salt Lake City, Utah 84121 (US)**

(72) Inventor : **Beplate, Douglas K.**
**6688 Royal Harvest Way No. 11**
**Salt Lake City, Utah 84121 (US)**

(74) Representative : **W.P. Thompson & Co.**
**Coopers Building, Church Street**
**Liverpool L1 3AB (GB)**

(54) **Diaper.**

(57) A diaper includes an absorbent pad (40) inserted in an opening in an inner panty (30), the inner panty holding the absorbent pad snugly between the legs of the wearer. An outer panty (12) is secured around its periphery to the inner panty and encloses the same in spaced relationship. The spaced relationship (13) forms an overflow reservoir for the absorbent pad (40).

FIG. 3

EP 0 498 612 A1

This invention relates to diapers and, more particularly, to a reusable diaper and method of making same.

Diapers of one form or another have been known for many generations and are generally defined as a basic garment for infants and incontinent adults. A conventional diaper consists of a folded cloth or other absorbent material drawn up between the legs and fastened about the waist. Historically, diapers were available in the form of a layer of cloth about one metre square. To produce a suitable diaper, the cloth was folded in any one of a plurality of patterns to achieve the appropriate diaper size and then pinned with a safety pin about the waist of the wearer. This entire process is fraught with problems not only in folding the diaper to the wrong size but also in injuries resulting from accidental punctures from the safety pin. Since the primary function of the diaper is to absorb urine and act as a catchment for faeces, considerable effort has been made to reduce, or even eliminate, the natural revulsion one feels when required to change a diaper, particularly one containing faeces. Cloth diapers also require the use of a separate, water-repellent cover to resist leakage of urine or even watery faeces through the cloth fabric. The result of the foregoing is that, within the past few decades there has been an explosive increase in the use of disposable diapers in both the paediatric and the adult settings. User convenience along with the aesthetics of disposability have been the primary driving forces behind the wide acceptance of disposable diapers.

While convenient, disposable diapers represent not only a significant increase in cost, but, more importantly, represent a major concern environmentally in that they constitute a significant portion of the solid waste stream. This, in turn, means that a significant portion of the landfill space is occupied by disposable diapers. Further, since a significant number of the disposable diapers contain faeces, they also represent a threat to the environment through fecal contamination, particularly due to the pathogens carried in most faeces. One of the principal advantages to the use of cloth diapers is the fact that the human wastes are directed into the sewer system.

Clearly, each prior art system of diapers has its advantages and disadvantages. Accordingly, it would be a significant advancement in the art to provide a diaper that incorporates the advantages from each system. It would also be an advancement in the art to provide a reusable diaper that is prefolded and includes a water-resistant outer shell. Another advancement would be to provide a reusable diaper having a removable liner to facilitate transfer of faeces from the diaper to the toilet or other waste disposal facility.

This invention provides a reusable diaper configured with an absorbent, cloth-covered pad incorporated as an integral unit in an inner shell, the inner shell being enclosed in an outer, water-resistant shell. The inner shell suspends the absorbent pad snugly between the legs of the wearer and in spaced relationship to the outer shell. The inner shell in the region of the absorbent pad is in fluid communication with the spatial separation between the inner shell and the outer shell to allow surplus liquid to pass into this space. The inner shell also supports the absorbent pad in the extended configuration to resist its bunching during periods of wear. An optional liner may be made available to facilitate removal of any faeces deposited in the diaper. The liner can be either reusable or disposable. A hook and hoop fastener system fastens the diaper about the waist of the wearer. Elasticised sections at each side of the diaper provide a snug fit around the legs of the wearer.

It is, therefore, one object of this invention to provide improvements in reusable diapers and in their manufacture.

Another object of this invention is to provide a reusable diaper characterised by the absence of absorbent material on the external profile of the legs of the wearer.

Another object of this invention is to provide improvements in the method of providing a diaper.

Another object of this invention is to provide a reusable diaper having an absorbent pad incorporated into an inner shell with the inner shell enclosed in a water-resistant, outer shell.

Another object of this invention is to provide a reusable diaper having a removable liner to facilitate removal and disposal of faeces collected in the diaper.

Another object of this invention is to provide a reusable diaper having an absorbent pad supported snugly between the legs of a wearer, the absorbent pad being held against twisting or bunching while being held snugly between the legs of the wearer.

Another object of this invention is to provide a reusable diaper having a hook and loop fastener system for adjustably fastening the diaper about the waist of the wearer.

Diapers are important not only for the paediatric population but also for certain segments of the adult population. The term "diaper" is used herein in a generic sense for any absorbent-type undergarment worn for the catchment and containment of urine and/or faeces. The need for diapers among the paediatric population is accepted knowledge and is due to the fact that, in practically all cases of paediatric diaper usage, the diaper is a temporary (up to three or four years) measure until the wearer's physiological maturity progresses sufficiently to the point where the normal excretory functions can be controlled voluntarily. The term "paediatric population" is usually understood to mean those persons up to about three or four years of chronological age and a weight up to about 18 kg (40 pounds). The term "adult population" is used herein to describe all other persons who may require the use of a diaper either in an acute sense or a chronic sense.

Usage of diapers by the adult population is generally the result of enuresis, injuries, mental and/or physical deterioration, disease, confinement, incontinence, and the like, regardless of the origin of the particular problem. For instance, many women suffer from certain forms of urinary incontinence due to injuries inflicted on the bladder sphincter during childbirth. Physical incapacity as well as mental dementia, particularly among the geriatric portion of the adult population, appears to be the major factor necessitating the use of diapers among this population. In either circumstance, it is important for the wearer that the diaper should be easily affixed either by the wearer or another person and changeable with equal facility.

Advantageously, the diaper of this invention is configured with an absorbent pad that is held snugly in place between the legs and is particularly characterised by the absence of padding on the outside of the profile of the legs. This means that, unlike prior art reusable diapers, there is no extraneous bulk around the waist or legs of the wearer to reveal to the casual observer that the wearer is wearing a diaper. Not only does this feature enable the ambulatory wearer to wear the diaper of this invention under normal clothing but it also significantly enhances the self esteem of the wearer by the knowledge that the presence of a diaper on the wearer is effectively hidden from accidental discovery or observation. The reusable diaper system of this invention also eliminates embarrassment from the presence of a disposable diaper in the garbage system where it could be observed by others to the shame and embarrassment of the wearer.

The invention will be further described, by way of example, with reference to the accompanying drawings, in which:

Fig.1 is a perspective view of a preferred embodiment of diaper of this invention;

Fig.2 is a plan view of the diaper but with the diaper opened;

Fig.3 is a cross-sectional view taken along lines 3-3 of Fig.2;

Fig.4 is an exploded plan view of the various elements that are assembled into the diaper shown in Fig.1; and

Fig.5 is a plan view of a removable liner for the reusable diaper of this invention.

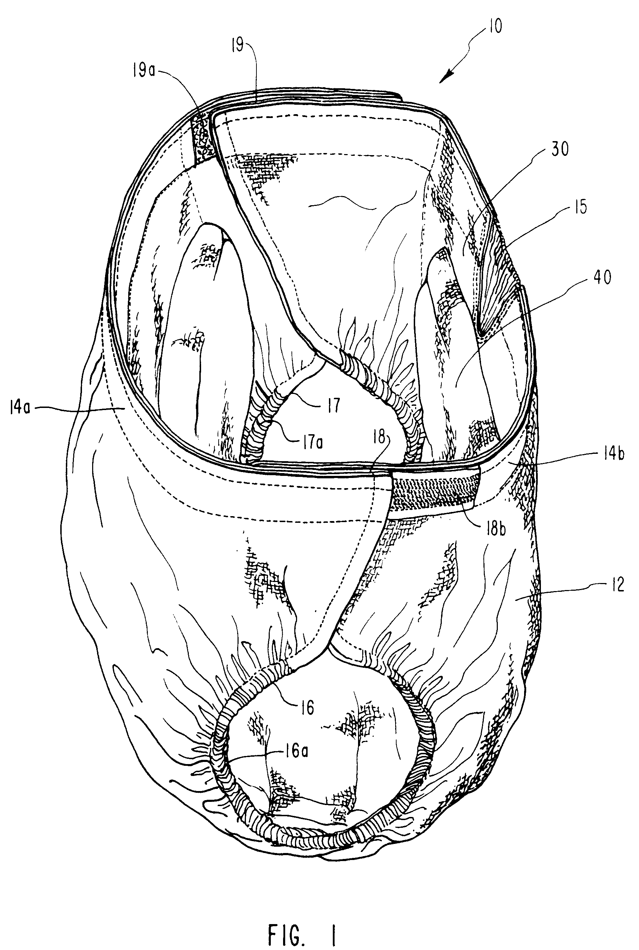

Referring now to Fig. 1, a reusable diaper 10 of this invention includes an outer panty or shell 12 and an inner panty or shell 30 with the inner shell 30 supporting an absorbent pad 40 in spaced relationship inside the outer shell 12. Reusable diaper 10 is configured with a panty-like external profile with a waist band separated into a front waist band 14a and a rear waist band 14b. Front waist band 14a is configured to be releasably joined to rear waist band 14b at each side of reusable diaper 10 above leg openings 16 and 17. Leg opening 16 is designed as an opening from the left leg of a wearer (not shown) and includes an elasticised segment 16a to assure a snug fit about the leg (not shown) while leg opening 17 is correspondingly configured with an elasticised segment 17a to accommodate the right leg of the wearer (not shown) in a snug-fitting relationship.

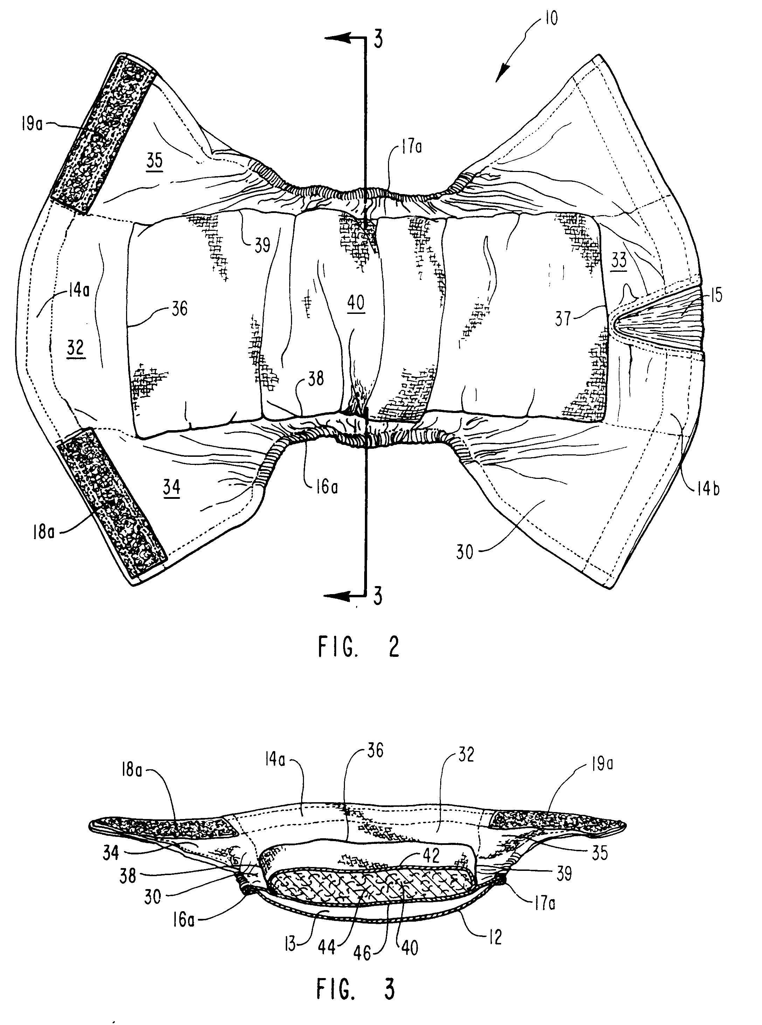

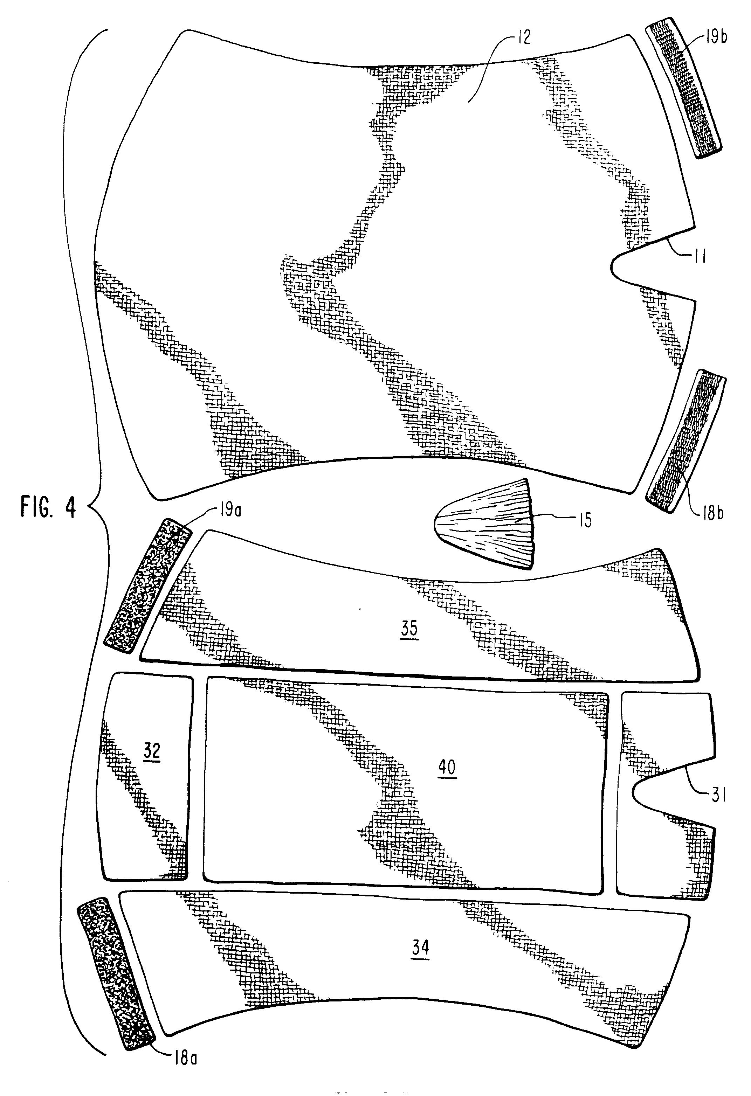

The releasable joinder of the ends of front waist band 14a to the respective ends of rear waist band 14b is accomplished using matching pairs of hook and loop fastener systems 18 and 19. Loop portions 8a and 19a of hook and loop fastener systems 18 and 19, respectively, are attached at each end of front waist band 14a while hook portion 18b and hook portion 19b (Fig.4) are attached at each end an on the outside face of rear waist band 14b. This particular orientation of the respective hook and loop portions of hook and loop fasteners 18 and 19 is important due to the inherent nature of commercially available hook and loop fastener systems. In particular, the loop portion is generally configured with a relatively soft, felt-like texture whereas the hook portion is specifically designed with a certain degree of stiffness to enable the hooks therein to suitably penetrate the loops so as to releasably engage the same. Such hook and loop fastener systems are widely available commercially from Velcro, Inc., Manchester, New Hampshire, under their trademark VELCRO. In view of the relatively soft, felt-like texture of loop portions 18a (Figs. 2 to 4) and 19a, they are placed on the inner face of front waist band 14a where any exposed portions thereof (as shown in Fig.1 by loop portion 19a) are placed in contact with the wearer (not shown). It is particularly important that hook portions 18b and 19b (Fig.4) are placed on the outside face of rear waist band 14b so as to minimise contact by the wearer (not shown).

Even though reusable diaper 10 is intended to be fully reusable, the same features can be incorporated, advantageously, into a diaper 10 that is entirely disposable. As such, diaper 10 provides significant advantages in that the total bulk thereof as the result of the overall size and placement of absorbent pad 40 is substantially reduced as compared with a commercially available, disposable diaper (not shown). In particular, absorbent pad 40 as well as inner shell 30 and outer shell 14 can be fabricated entirely from materials acceptable as solid wastes and, as such, would provide significant advantages since the overall bulk of absorbent pad 40 is substantially less than the conventional, commercially available, disposable diaper (not shown).

The back of reusable diaper 10 includes an elastic gore 15 of an elastic fabric inserted in the centre of rear waist band 14b. Elastic gore 15 is designed to enhance the fit of waist band 14 about the waist of a wearer (not shown) by imparting a limited degree of elasticity to waist band 14. This amount of elasticity is sufficient to adapt waist band 14 to changes to the circumference of waist of the wearer (not shown) during movement, changes in posture, breathing, and the like.

Referring now also to Fig.2, inner shell 30 substantially conforms to the external profile of outer shell 12 but is assembled from a front panel 32, a rear panel 33, a left panel 34, and a right panel 35. Front panel 32 is joined to a front end of absorbent pad 40 along a seam 36 while rear panel 33 is joined to a rear end of absorbent pad 40 along a seam 37. Left Panel 34 extends the full length of inner shell 30 and is joined along a seam 38 to a left edge of each of front panel 32, absorbent pad 40, and rear panel 33. Similarly, right panel 35 extends the full length of inner shell 30 and is joined along a seam 39 to the right side of each of front panel 32, absorbent pad 40, and rear panel 33.

Inner shell 30 is designed to suspend absorbent pad 40 in spaced relationship between leg openings 16 and 17 (Figure 1) and thereby suspend absorbent pad 40 snugly between the legs of the wearer (not shown) when waist band 14a,14b is snugly engaged around the waist of the same. Further, inner shell 30 is also specifically configured to suspend absorbent pad 40 in spaced relationship within the profile of outer shell 12. With particular reference also to Fig.3, absorbent pad 40 is suspended by inner shell 30 in spaced relationship to outer shell 12, the spatial separation therebetween being shown as spatial separation 13.

Absorbent pad 40 is configured from an upper layer 42 and a lower layer 46 with a fibrous fill 44 therebetween. Upper layer 42 and lower layer 46 are fabricated from a soft fabric material, such as a cotton flannel while fibrous fill 44 is selected from a nonwoven, batting-type material, such as a polyester, or the like. In one presently preferred embodiment fibrous fill 44 was a blended cotton and wool batting. In effect, absorbent pad 40 is constructed as a small quilt or pillow whose primary function is the absorption and retention of liquids. Upper layer 42 is specifically directed to a soft, absorbent, non-allergenic material, such as cotton flannel, since its primary function is to reside snugly between the legs of a wearer and wick away any moisture deposited thereon. The moisture (not shown) is pulled directly into fibre fill 44 through this inherent wick action.

Spatial separation 13 allows outer shell 12 to assume a loose, slightly bouffant profile when secured to a wearer with the additional advantage of forming an overflow reservoir in the event excess liquid is deposited in absorbent pad 40. However, given the nature of absorbent pad 40 this eventuality is somewhat limited. For example, in one experimental test, over 280 millilitres of water were poured on and absorbed by absorbent pad 40 without any of the water passing into spatial separation 13. This particular experiment was conducted using a paediatric size, reusable diaper 10. The advantage of absorbent pad 40 in such a circumstance is that a paediatric wearer (not shown) of reusable diaper 10 has normal bladder capacity of only about 85 millilitres.

Absorbent pad 40 performs another function when reusable diaper 10 is used in an adult setting. In particular, for those instances of bladder incontinence, the outflow of urine is more or less a constant drip generally at a rate that approximates the excretion of urine from the kidneys. In such circumstances, it is highly desirable for upper layer 42 to wick away this liquid directly into fibre fill 44. Absorbent pad 40 thereby quickly and efficiently retains the absorbed liquid while inner shell 30 suspends absorbent pad between leg openings 16,17. This feature is important since it effectively inhibits excess liquid in absorbent pad 40 from leaking out of either of leg openings 16 or 17.

Outer shell 12 and inner shell 30 are each fabricated from a water resistant fabric, such as a nylon. This is important not only with respect to outer shell 12 and the fact that it creates an overflow reservoir in spatial separation 13, but because it also effectively inhibits the migration or wick action of liquid from absorbent pad 40 through either left panel 34 or right panel 35 to the respective leg opening 16 or 17.

With reference now to Fig.4, outer shell 12 includes a cutout 11 adapted to receive elastic gore 15. A matching cutout 31 is also found in rear panel 33 and, when superimposed over cutout 11, conforms to the profile of elastic gore 15.

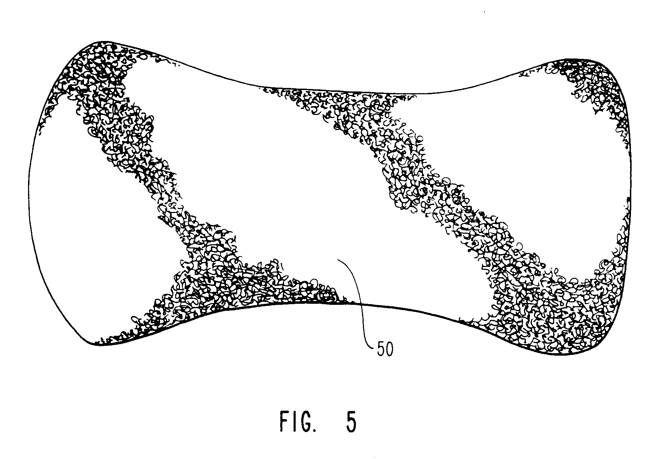

Fig.5 shows a liner 50 that is adapted to be placed on top of absorbent pad 40 for the purpose of acting as a catchment for the solids part of faeces excreted by the wearer (not shown) of reusable diaper 10. Liner 50 may be fabricated from a flannel cloth material with the intention of being either reusable or even disposable. As a reusable item, liner 50 significantly reduces handling problems when faeces (not shown) is deposited thereon since it is a simple matter for the attendant (not shown) to simply grasp each end of liner 50 and transport it to the appropriate waste receptacle (not shown). As a disposable system, liner 50 is fabricated from a suitable fabric material commonly found in disposable diapers, for example, and deposited directly into a toilet for disposal.

In the method of making a reusable diaper, the reusable diaper 10 is assembled with inner shell 30 enclosed within outer shell 12. Absorbent pad 40 is suspended in inner shell 30 and is specially configured to be held snugly between the legs of the wearer (not shown). Inner shell 30 is configured with a modified hour glass-like outline as is outer shell 12 so as to readily adapt reusable diaper 10 to being worn between the legs of the wearer (not shown). Inner shell 30 is joined to outer shell 12 along their respective external perimeters so as to effectively enclose absorbent pad 40 inside the confines of outer shell 12. Importantly, absorbent pad 40 is not merely attached to an upper surface of inner shell 12 but is, in effect, inserted in an opening formed therein through the joinder of front panel 32 and rear panel 33 with each of left panel 34 and right panel 35. In this manner, excess liquid (not shown) received by absorbent pad is free to enter spatial separation 13, thereby significantly reducing the possibility that the excess liquid could escape from either of leg openings 16 and 17. Clearly, if absorbent pad 40 were placed directly on top of a water-resistant fabric (such as if inner shell 30 were constructed similarly to outer shell 12) there would be a very high probability that the excess liquid would leak out of either of leg openings 16 and 17.

Reusable diaper 10 is readily mounted and removed from about the waist of the wearer (not shown). Mounting is accomplished by bringing absorbent pad 40 snugly between the legs and fastening waist band 14a,14b about the waist. Hook and loop fasteners 18 and 19 each have sufficient length to accommodate adjustably securing waist band 14a,14b. Further, elastic gore 15 contributes a limited degree of elasticity to waist band 14a,14b to accommodate changes in the circumference of waist band 14a,14b during wear of reusable diaper 10.

Advantageously, since all of the bulk of absorbent pad 40 is held between the legs, the only visible portion of reusable diaper 10 on the outsides of the legs are the respective portions of inner shell 30 and outer shell 12. Accordingly, excessive bulk is utterly eliminated from those portions of reusable diaper 10 which would otherwise create an unsightly bulge in the outer clothing of the wearer. This is important particularly when reusable diaper 10 is configured as an undergarment for a member of the adult population.

Reusable diaper 10 is easily replaced by simply separating hook and loop fasteners 18 and 19 and removing absorbent pad 40 from between the legs of the wearer. Since the total bulk of reusable diaper 10 is substantially smaller than that of a conventional disposable diaper as well as a conventional reusable diaper, reusable diaper 10 is readily concealable (if necessary) for transport to a place for washing the same.

**Claims**

1. A diaper comprising an absorbent pad (40) and a support (12,30) for supporting the pad (40) at a position between the legs of a wearer; characterised in that the support comprises an inner support shell (30) for supporting said absorbent pad (40) and attached to said pad (40) at the periphery thereof and extending outwardly therefrom, said inner support shell (30) having a profile substantially conforming to a modified hourglass configuration and being adapted to be attached about the wearer's waist; and an outer shell (12) secured to said inner support shell (30) and forming an outer cover for said absorbent pad (40).

2. A diaper comprising a panty and an absorbent pad (40) secured to the panty; characterised in that the panty comprises an outer shell (12) including a front panel and a back panel with a crotch panel adapted to be worn between the legs of a wearer, the outer shell (12) having a waist band in two segments, namely a front waist band (14a) and a rear waistband (14b), said waist band including hook and loop fastener means (18,19) for adjustably securing said waist band segments together; and an inner shell (30) secured to said outer shell (12) along the peripheries of said inner and outer shells, said inner shell (30) being suspended inside and spaced from said outer shell (12); the inner shell (30) having an elongated opening and the external periphery of said pad (40) corresponding to said elongated opening, said pad (40) being secured along its external periphery in said elongated opening in said inner shell (30).

3. A diaper as claimed in claim 1 or 2, wherein said absorbent pad is prepared from an upper layer (42) of fabric or cloth and a lower layer (46) of fabric or cloth with a layer of absorbent fibrous material or batting (44) therebetween.

4. A diaper as claimed in claim 3, wherein said upper layer of cloth, said lower layer of cloth, and said fibrous batting are selected from reusable materials so as to provide a reusable diaper.

5. A diaper as claimed in any of claims 1 to 4, wherein said inner support shell (30) comprises two side panels (34,35) along each side of said absorbent pad (40), said side panels (34,35) extending beyond the ends of said pad (40), said inner support shell also including a front panel (32) and a rear panel (33), said front panel (32) and said rear panel (33) each extending between said side panels (34,35).

6. A diaper as claimed in any of claims 1 to 4, wherein said inner shell (30) comprises a right panel (34) sewn to a right edge of said absorbent pad (40), a left panel (35) sewn to a left edge of said pad, a front panel (32) sewn to a front edge of said pad, and a rear panel (33) sewn to a rear edge of said pad.

7. A diaper as claimed in claim 5 or 6, wherein an outer edge of each side panel (34,35) includes a length of elastic (16a,17a) to adapt said side panels into conformance around the legs of the wearer.

8. A diaper as claimed in any of claims 1 to 7, which includes a waist band in two segments (14a,14b) which are provided with hook and loop fasteners (18,19) to accommodate adjustably attaching said diaper about the wearer's waist.

9. A diaper as claimed in any of claims 1 to 8, which is provided with a removable liner (50) releasably mounted in said diaper at a position superimposed over said absorbent pad (40).

10. A diaper as claimed in any of claims 1 to 9, wherein said outer shell (12) is fabricated from a moisture-resistant fabric.

11. A diaper as claimed in any of claims 1 to 7, wherein said absorbent pad (40), said inner support shell (30), and said outer shell (12) are constructed of fabric so that the diaper is a reusable diaper.

12. A diaper as claimed in any of claims 1 to 11, wherein said absorbent pad (40) is dimensionally configured to reside between the legs of the wearer and the diaper is particularly distinguished by the absence of absorbent pad (40) at an external profile outside the legs of the wearer.

13. A method for providing a diaper for a person comprising:
    preparing an absorbent pad to be worn between the legs of said person, said absorbent pad including an upper fabric layer, a lower fabric layer, and a fibrous fill between said upper fabric layer and said lower fabric layer;
    configuring an inner shell to be worn as an inner panty by said person, said inner shell having an elongated opening having dimensions corresponding to said absorbent pad;
    mounting said absorbent pad in said elongated opening in said inner shell;
    enclosing said inner shell in an outer shell, said outer shell being configured to be worn as outer panty by said person; and
    attaching said inner panty and said outer panty about the waist of said person, said inner panty supporting said absorbent pad snugly between said legs of said person.

14. A method as claimed in claim 13, wherein said enclosing step includes forming a surplus liquid reservoir below said absorbent pad by holding said outer panty in spaced relationship to said inner panty and said absorbent pad.

15. A method as claimed in claim 13 or 14, wherein said preparing step includes limiting the external periphery of said absorbent pad so as to preclude said absorbent pad from extending outwardly to the sides of said legs.

FIG. I

FIG. 2

FIG. 3

FIG. 4

FIG. 5

European Patent
Office

## EUROPEAN SEARCH REPORT

Application Number

EP    92 30 0925

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-9 007 313 (WORLWIDE BABY BASICS INFANT WEAR LTD) <br> * the whole document * | 1-11, 13-15 | A61F13/62 |
| X | EP-A-0 194 453 (EXQUISIT KURT GOETZ) <br><br> * claims; figures * | 1-3,5-8, 10,13-15 | |
| P,X | EP-A-0 433 951 (KIMBERLAY CLARK CO) | 1,2,8, 10,12 | |
| P,A | <br> * the whole document * | 3,13 | |
| P,X | EP-A-0 430 443 (ARCO CHEMICAL TECHNOLOGY INC) <br><br> * claims * | 1,3,4, 10-14 | |
| A | US-A-3 882 871 (TAKASI TANIGUCHI) <br> * the whole document * | 1-15 | |
| A | DE-A-3 533 881 (E. GILOMEN) <br> * claims; figures * | 1-15 | TECHNICAL FIELDS SEARCHED (Int. Cl.5 ) <br><br> A61F |

The present search report has been drawn up for all claims

| Place of search | Date of completion of the search | Examiner |
|---|---|---|
| THE HAGUE | 08 APRIL 1992 | M. VANMOL |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)